# EUROPEAN PATENT SPECIFICATION

(11) **EP 2 303 228 B1**
(45) Date of publication and mention of the grant of the patent: **30.11.2011**
(21) Application number: 09749606.1
(22) Date of filing: 19.05.2009
(51) Int. Cl.: A61K 9/08, A61K 31/675

(54) **FOSPHENYTOIN COMPOSITION**
FOSPHENYTOIN-ZUSAMMENSETZUNG
COMPOSITION DE FOSPHÉNYTOÏNE

(30) Priority: 19.05.2008 US 128266 P
(43) Date of publication of application: 06.04.2011
(73) Proprietor: Texcontor Etablissement, 9490 Vaduz (LI)
(72) Inventor: TRUONG, Van Hung, Westerville, Ohio 43082 (US)
(74) Representative: Campbell, Neil Boyd
(86) International application number: PCT/EP2009/003563
(87) International publication number: WO 2009/141116

(56) References cited:
- WO-A-98/56422
- US-A- 6 133 248
- US-A1- 2007 249 563
- STELLA ET AL: "Prodrug strategies to overcome poor water solubility" 24 August 2007 (2007-08-24), ADVANCED DRUG DELIVERY REVIEWS, ELSEVIER BV, AMSTERDAM, NL, PAGE(S) 677 - 694 , XP022211987 ISSN: 0169-409X page 688, left-hand column, line 19 - page 689, left-hand column, line 7

## Description

This invention relates to aqueous pharmaceutical compositions and to the application of such compositions as anti-convulsant agents. In particular, the invention is concerned with aqueous compositions comprising 5,5-diphenyl-3-[(phosphonooxy) methyl]-2,4-imidazolidinedione (fosphenytoin) or a salt thereof according to claim 1 which are stable at room temperature.

5,5-diphenyl-3-[(phosphonooxy) methyl]-2,4-imidazolidinedione, more commonly known as fosphenytoin, is a prodrug of 5,5'-diphenylhydantoin. 5,5-diphenylhyantoin, hereinafter referred to by its trivial name phenytoin, is commonly used in the treatment and management of convulsive states (e.g. epilepsy and seizures). Its application in the treatment of such states means that it is often necessary for phenytoin to be administered parenterally, often intravenously or intramuscularly, rather than orally. Phenytoin, however, is practically insoluble in water and therefore has a low bioavailability. Phenytoin therefore usually has to be dissolved in an aqueous alkaline medium of pH 12 for administration. Use of such an alkaline pH can, however, cause irritation.

Moreover, the use of an alkaline solution does not completely alleviate the problems associated with the phenytoin. The low water solubility of phenytoin, for example, means that it has a tendency to precipitate during and shortly after delivery making its parenteral administration painful. In fact it has even been reported that precipitation during intramuscular delivery can cause tissue damage and/or haemorrhage, haematoma and necrosis at the injection site. Precipitation during and/or after administration can also lead to less than the intended dose being delivered (e.g. if precipitated phenytoin is left in the needle) as well as to delayed release of phenytoin and/or erratic phenytoin blood levels.

To circumvent the problems associated with use of phenytoin several prodrugs have been developed (US-A-4,260,769). One pro-drug which has been found to be particularly useful is fosphenytoin sodium, which is the disodium salt of the phosphate ester of the hydroxymethyl derivative of phenytoin. This prodrug is sold by Parke-Davis under the trademark Cerebyx^{®}. Cerebyx^{®} comprises a ready mixed solution of 75 mg/ml fosphenytoin sodium in water and tromethamine buffer adjusted to pH 8.6 to 9.0 with either hydrochloric acid or sodium hydroxide. It is recommended that the vials containing the Cerebux^{®} solution are stored in a refrigerator between 2-8 °C away from light and moisture. Such storage at low temperatures helps to prevent degradation of the fosphenytoin disodium salt to phenytoin and other degradation products such as carbamoyl diphenyl glycine.

US-A-4,925,860 describes an investigation into the degradation pathway of fosphenytoin to phenytoin and hypothesizes that the pathway proceeds with an overall consumption of hydroxyl and via formation of one or more of formaldehyde, 5,5-diphenyl-4-imidazolidinone (DIZ), diphenylglycinamide, diphenylglycine and benzophenone. In light of the proposed consumption of hydroxyl, US-A-4,925,860 advocates addition of a buffer, which is capable of preventing the pH from dropping, to affect stabilisation.

US-A-4,925,860 further teaches that the degradation pathway changes as pH drops and that a high pH should be maintained to ensure that low solubility phenytoin is not the main degradation product. Accordingly, US-A-4,925,860 teaches use of an aqueous formulation comprising fosphenytoin and a 0.05 to 0.2 M buffer wherein the pH of the system is maintained between 8.3 and 9.4.

Hence the pH range of 8.3 to 9.4 in US-A-4,925,860 and the pH of 8.6 to 9.0 in Cerebyx^{®} aims to ensure that the minimum quantities of phenytoin are produced during storage of the fosphenytoin solution. On the other hand, however, significant amounts of other degradation products may be produced. In fact US-A-4,925,860 specifically aims to optimize the pH range such that the primary degradation product is the compound it identifies as diphenylglycinamide, rather than to eliminate degradation *per se.* Thus, as shown by the results in US-4,925,860, aqueous solutions comprising fosphenytoin and buffer (0.05 to 0.2 M), and having a pH between 8.3 and 9.4, tend to undergo degradation when stored at room temperature. The main degradation product is the compound identified in US-4,925,860 as diphenylglycinamide, though formaldehyde is also often produced in significant amounts. These compounds, along with lesser amounts of, phenytoin, diphenylglycine, benzophenone and DIZ, may be present in a total amount of up to approximately 15 % by weight in a solution stored at room temperature or higher for at least 7 months. The amount of fosphenytoin, and therefore phenytoin, available for delivery after storage may therefore be significantly reduced. This not only means that less than the recommended dose of fosphenytoin, and therefore phenytoin, may ultimately be delivered to a patient but also that the exact amount of fosphenytoin, and therefore phenytoin, given is unknown.

WO9856422, US6133248 and Stella at al. Prodrug strategies to overcome poor water solubility. Advanced Drug Delivery Reviews. 24.08.2007 disclose fosphenytoin compositions on the basis of cyclodextrin. US2007249563 discusses the prevention of degradation during manufacturing of fosphenytoin and of derivatives thereof.

A need therefore remains for alternative compositions comprising fosphenytoin and in particular for compositions which are stable (e.g. can be stored at room temperature without undergoing degradation) and which can be safely and painlessly administered by the parenteral route. It has now been surprisingly found that an aqueous composition comprising fosphenytoin buffers selected from sodium bicarbonate, sodium phosphate, boric acid, glycine, and having a pH of less than 8.3 is stable for prolonged periods of time even at room temperature. In particular it has been found that such compositions are resistant to degradation and produce only small amounts, if any, of phenytoin or any of the other above-mentioned degradation products when stored at room temperature. This means that the aqueous compositions of the invention may be used to painlessly and reliably, administer a known dose of fosphenytoin, and therefore, phenytoin, even after prolonged storage of the composition.

Thus viewed from one aspect the invention provides an aqueous pharmaceutical composition comprising 5,5-diphenyl-3-[(phosphonooxy)methyl]-2,4-imidazolidinedione or a salt thereof, wherein said composition has a pH of less than 8.3

Viewed from a further aspect the invention provides a method of treatment of a human or non-human animal subject in need of anti-convulsant treatment, said method comprising administering to said subject an effective amount of an aqueous pharmaceutical composition comprising 5,5-diphenyl-3-[(phosphonooxy)methyl]-2,4-imidazolidinedione or a salt thereof, wherein said composition has a pH of less than 8.3.

5,5-diphenyl-3-[(phosphonooxy)methyl]-2,4-imidazolidinedione and salts thereof for use according to the invention may be obtained from commercial sources or may be prepared using standard processes and procedures well known to those skilled in the art. 5,5-diphenyl-3-[(phosphonooxy)methyl]-2,4-imidazolidinedione has the formula:

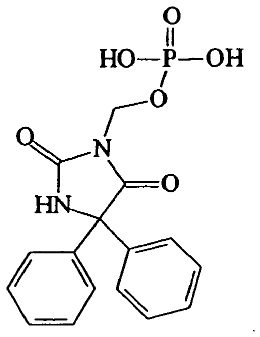

5,5-diphenyl-3-[(phosphonooxy)methyl]-2,4-imidazolidinedione is also known as 3-(hydroxymethyl)-5,5-diphenylhydantoin phosphate ester, 2,5-dioxo-4,4-diphenylimidazolidin-1-yl-methyl phosphate and, as mentioned above, fosphenytoin. The term 5,5-diphenyl-3-[(phosphonooxy)methyl]-2,4-imidazolidinedione is used herein synonymously with all of these terms.

In preferred compositions of the invention the 5,5-diphenyl-3-[(phosphonooxy)methyl]-2,4-imidazolidinedione or salt thereof used is of pharmaceutical grade purity. Typically 5,5-diphenyl-3-[(phosphonooxy)methyl]-2,4-imidazolidinedione or a salt thereof for use in the invention contains 0.1 % wt or less phenytoin, more preferably 0.05 % wt or less phenytoin, more preferably 0.02 % wt or less phenytoin, e.g. about 0.01 % wt or less phenytoin. The amount of phenytoin present in 5,5-diphenyl-3-[(phosphonooxy)methyl]-2,4-imidazolidinedione or a salt thereof may, for example, be determined by the chromatographic assay procedure described in United States Pharmacopeia 27, Supplement I.

The phrase "wherein said composition has a pH of less than 8.3" as used herein is intended to cover those compositions which have a pH of less than 8.3 immediately after its preparation (e.g. following sterilisation). The pH of the composition may subsequently change (e.g. fall) during storage. Similarly it is intended that any pH range specified herein covers those compositions possessing a pH which falls within the specified range immediately after its preparation (e.g. following sterilisation).

In preferred compositions of the invention the pH is greater than 7.5. In other words the pH of the composition is greater than 7.5 and less than 8.3 (i.e. 7.5 > pH < 8.3). More preferably the pH of the composition is 8.2 or less, still more preferably 8.1 or less, e.g 8.0 or less. In other words the pH of the composition is greater than 7.5 and less than or equal to 8.2, greater than 7.5 and less than or equal to 8.1 and greater than 7.5 and less than or equal to pH 8.0 respectively. In particularly preferred compositions of the invention the pH of the composition is 7.6 or greater, still more preferably 7.7 or greater, e.g. 7.8 or greater. In other words the pH of the composition is greater than or equal to 7.6 and less than 8.3, greater than or equal to 7.7 and less than 8.3 and greater than or equal to 7.8 and less than 8.3 respectively. Representative examples of the pH range of the composition of the invention are: (i) 7.5 to 8.2, (ii) 7.7 to 8.2, (iii) 7.5 to 8.1, (iv) 7.7 to 8.1, (v) 8.0 to less than 8.3, e.g. 8.0 to 8.2.

In preferred compositions of the invention the aqueous composition comprises water for injections. Still more preferably the water present in the composition of the invention consists essentially of (e.g. consists of) water for injections.

Preferably the composition of the invention also comprises a pharmaceutically acceptable buffer. Any buffer which is compatible with 5,5-diphenyl-3-[(phosphonooxy)methyl]-2,4-imidazolidinedione or a salt thereof and is effective to maintain the required pH range may be used. For example, buffers comprising an organic or inorganic acid having a pKa in the range 7.2 to 10.2 may be used. Representative examples of suitable buffers include those comprising a weak acid selected from boric acid, carbonic acid, phosphoric acid, amino acids (e.g. alanine, glycine, aspartic acid, cysteine, histidine, lysine), amino acid derivatives (e.g. dimethylglycine), allantoin and ascorbic acid.

Preferred buffers include those which comprise a weak acid selected from carbonic acid, phosphoric acid, boric acid and glycine. Especially preferred buffers comprise a weak acid selected from boric acid, glycine and salts of bicarbonate (e.g. sodium bicarbonate) and phosphate (e.g. sodium phosphate). Still more preferred buffers comprise a weak acid selected from boric acid and glycine.

The buffer may be used at any concentration which is effective to provide buffering in the required pH range. Generally, the buffer concentration may be less than 5 % wt, more preferably less than 2 % wt, e.g. less than 1 % wt. Sodium phosphate may, for example, be used at a concentration of about 0.8 % wt. Boric acid and glycine buffers may, for example, be used at a concentration of about 0.3 % wt. On the other hand, sodium bicarbonate may be used, for example, at a concentration of less than 0.01 % wt.

Thus typically the buffer may be used at a concentration of from 0.0005 to 0.1 M, more preferably from 0.0006 to 0.06 M, for example, about 0.05 M. Sodium phosphate, boric acid and glycine buffers may, for example, be used at a concentration of about 0.05 or 0.04 M. Advantageously sodium bicarbonate may be used at a lower concentration (e.g. about 0.0006 M).

In preferred compositions of the invention 5,5-diphenyl-3-[(phosphonooxy)methyl]-2,4-imidazolidinedione is present in the form of a salt. Salts may be formed with any pharmaceutically acceptable base. Representative examples of suitable bases include organic bases and those derived from alkali or alkaline earth metals. Exemplary organic bases include triethylamine, pyridine, piperidine, morpholine and N-methylmorpholine. More preferably 5,5-diphenyl-3-[(phosphonooxy)methyl]-2,4-imidazolidinedione is present in the form of a salt deriving from an alkali or alkaline earth metal base. Examples of suitable alkali or alkaline earth metal bases include those comprising sodium, potassium, calcium and magnesium, especially sodium. More preferably 5,5-diphenyl-3-[(phosphonooxy)methyl]-2,4-imidazolidinedione is present in the form of its disodium salt.

The quantity of 5,5-diphenyl-3-[(phosphonooxy)methyl]-2,4-imidazolidinedione or a salt thereof present in the composition of the invention may be readily determined by those skilled in the art and will depend on several factors, including the planned administration route, the weight of the potential patient and the planned state to be treated. Generally, however, 5,5-diphenyl-3-[(phosphonooxy)methyl]-2,4-imidazolidinedione or a salt thereof may be present in amounts of from 35-200 mg per dosage form, more preferably 50-100 mg per dosage form, e.g. about 75 mg per dosage form. Thus typically 5,5-diphenyl-3-[(phosphonooxy)methyl]-2;4-imidazolidinedione or a salt thereof may be present in an amount which provides 25-135 mg phenytoin per dosage form, more preferably 35-35 mg phenytoin per dosage form, e.g. about 50 mg phenytoin per dosage form. A "dosage form" may be considered to be one quantum of medicine (e.g. per measured amount of liquid). 5,5-diphenyl-3-[(phosphonooxy)methyl]-2,4-imidazolidinedione or a salt thereof may be conveniently used in amounts of from 2.5-20 wt % of the composition, preferably 5-10 wt %.

The compositions of the invention may additionally comprise one or more further compounds. For instance, the compositions of the invention may additionally comprise one or more wetting agents and/or solubility enhancers. Examples of suitable wetting agents include polysorbate-80, poloxamer and polyoxyethylene fatty acid esters, benzalkonium chloride, benzethonium chloride, docusate sodium, polyoxyethylene stearates and sodium lauryl sulfate. Suitable solubility enhancers are compounds which increase the solubility of phenytoin in water. Examples of suitable agents include alcohol, propylene glycol, arginine (e.g. L-arginine), sodium desoxycholate, polysorbate-80, sulfobutylether-B-cyclodextrin and various combinations of these compounds.

Other compounds which may be present in the composition of the invention include one or more preserving agents and/or absorption enhancers. Moreover the composition of the invention may additionally comprise further (e.g. one or more) pharmaceutically active substances.

Particularly preferred compositions of the invention may have the following compositions:
(1) 5,5-diphenyl-3-[(phosphonooxy)methyl]-2,4-imidazolidinedione disodium salt (75 mg/ml), 0.052 M boric acid buffer, pH 7.8-8.2
(2) 5,5-diphenyl-3-[(phosphonooxy)methyl]-2,4-imidazolidinedione disodium salt (75 mg/ml), 0.050 M glycine buffer, pH 7.8-8.2

The compositions of the invention may be administered parenterally, i.e. by means other than through the gastrointestinal tract. Suitable means include intramuscular or intravenous injection. The composition may be presented in any form adapted for use in the administration route selected. Forms suitable for intramuscular or intravenous injection may, for example, be vials containing a pre-determined amount of the composition of the invention or pre-loaded syringes. Preferably the composition of the invention is contained in a syringe.

Thus viewed from a further aspect the invention provides a syringe containing 5,5-diphenyl-3-[(phosphonooxy)methyl]-2,4-imidazolidinedione or a salt thereof, wherein said composition has a pH of less than 8.3. All preferred features of the compositions hereinbefore described are also preferred features of composition-containing syringes.

The compositions of the invention may be prepared by any conventional method known in the art. Generally, however, the compositions of the invention are prepared by mixing the required amount of 5,5-diphenyl-3-[(phosphonooxy)methyl]-2,4-imidazolidinedione or a salt thereof with water and optionally (e.g. preferably) buffer, and sterilising the resulting mixture. An acid or base may also be added during preparation of the composition (e.g. before or after the addition of buffer) in order to adjust the pH to the desired range. Any pharmaceutically acceptable acid or base may be used for this purpose, e.g a suitable acid is HCl and a suitable base is NaOH.

During preparation of the compositions of the invention the mixing may take place in the container in which the composition will be stored (e.g. a vial or a syringe). Alternatively the composition may be first prepared then aliquoted into suitable containers (e.g. vials or syringes). Once in a container the composition is typically sterilised and the container sealed. Once sealed the container may be stored.

Storage may occur above room temperature (e.g. at 30°C or 40 °C), at room temperature (e.g. 25°C) or below room temperature (e.g. at 2-8 °C). Preferably the compositions of the invention are suitable for storage at 0 to 40 °C (e.g. 30 or 25°C, especially 25 °C). Still more preferably the compositions of the invention may be stored at room temperature for a prolonged period of time (e.g. at least 1 month, preferably at least 6 months, more preferably at least 12 months, e.g. up to 18 months). In particularly preferred compositions of the invention, the total amount of impurities produced by degradation of 5,5-diphenyl-3-[(phosphonooxy)methyl]-2,4-imidazolidinedione or a salt thereof (e.g. fosphenytoin disodium) during storage at 25 °C for 8 weeks is less than 0.3 wt %, more preferably less than 0.15 wt %, still more preferably less than 0.1 wt %, e.g. less than 0.7 wt %.

Thus viewed from a further aspect the invention provides a method of inhibiting (e.g. preventing) degradation of an aqueous solution of 5,5-diphenyl-3-[(phosphonooxy)methyl]-2,4-imidazolidinedione or a salt thereof comprising maintaining the pH of said solution at less than 8.3. More preferably the pH of the solution is as described hereinbefore in relation to the compositions of the invention. Still more preferably the solution further comprises a buffer as hereinbefore described in relation to the compositions of the invention.

The compositions hereinbefore described are suitable for use in medicine. Thus viewed from a yet further aspect the invention provides an aqueous pharmaceutical composition comprising 5,5-diphenyl-3-[(phosphonooxy)methyl]-2,4-imidazolidinedione or a salt thereof, wherein said composition has a pH of less than 8.3 for use in medicine.

In particular the invention provides compositions as hereinbefore described for the therapeutic or prophylactic treatment of convulsive states. A convulsive state may be considered to be any condition associated with uncontrolled contraction of the muscle parts of the body. Examples of convulsive states include generalized tonic-clonic seizures, complex partial seizures, seizures associated with neurosurgery and/or head trauma and convulsive status epilepticus and epilepsy. In particular the compositions of the invention may be used as part of the emergency treatment of a convulsive state (e.g. of status epilepticus) and for the prophylactic control of a convulsive state (e.g. seizures developing during or after neurosurgery or following traumatic injury to the head).

The compositions of the invention may also be suitable for the therapeutic or prophylactic treatment of cardiovascular disorders (e.g. cardiovascular complications associated with tricyclic antidepressant overdose, ventricular arrhythmias), trigeminal neuralgia, Wallenberg's syndrome and epidermolysis bullosa.

All preferred features of the compositions hereinbefore described are also preferred features of medicaments produced using 5,5-diphenyl-3-[(phosphonooxy)methyl]-2,4-imidazolidinedione or a salt thereof. Thus the present invention also provides use of 5,5-diphenyl-3-[(phosphonooxy)methyl]-2,4-imidazolidinedione or a salt thereof according to claim 1 in the manufacture of an aqueous pharmaceutical composition having a pH of less than 8.3 for treatment of any of the above-mentioned diseases.

The invention will now be described in more detail by way of the following non-limiting examples:

### EXAMPLES

Aqueous compositions comprising 75 mg/ml fosphenytoin disodium (containing ^{~}0.01 % wt phenytoin) were prepared using the buffers shown in Table 1. The compositions were prepared by making up the appropriate buffer (e.g dissolving the appropriate compound in water and diluting to the desired concentration) and adding fosphenytoin disodium. The pH of the resulting solution was then adjusted using hydrochloric acid and/or sodium hydroxide.

The compositions comprising sodium phosphate, sodium bicarbonate, boric acid and glycine but having a target pH of 9.9 are comparative examples (C1-C5).

The composition comprising tromethine buffer (C6) is a comparative example which represents a composition described by US-A-4,925,860.

**Table 1: Aqueous Compositions**

| Example No. | Buffer (M) | Target pH |
|---|---|---|
| 1 | None | 8.0 |
| 2 | 0.76 % Sodium phosphate, dibasic buffer (0.054 M) | 8.0 |
| 3 | 0.005 % Sodium bicarbonate, dibasic buffer (0.0006 M) | 8.0 |
| 4 | 0.319 % Boric acid buffer (0.052 M) | 8.0 |
| 5 | 0.376 % Glycine buffer (0.050 M) | 8.0 |
| C1 | None | 9.9 |
| C2 | 0.76 % Sodium phosphate, dibasic buffer (0.054 M) | 9.9 |
| C3 | 0.005 % Sodium bicarbonate, dibasic buffer (0.0006 M) | 9.9 |
| C4 | 0.319 % Boric acid buffer (0.052 M) | 9.9 |
| C5 | 0.376 % Glycine buffer (0.050 M) | 9.9 |
| C6 | Tromethamine buffer (0.05-0.2 M) | 8.9 |

Each of these compositions were stored under elevated temperature conditions (i.e. 25, 30 and 40 °C) for up to 8 weeks. At biweekly intervals the samples were evaluated for changes in product potency, impurity levels and pH. Evaluation was carried out using the chromatographic assay procedure described in United States Pharmacopoeia 27, Supplement I.

Additionally commercially available Cerebyx^{®} was also stored and analysed.

The results of the stability study are shown in the following tables wherein the following abbreviations are used: RRT = retention time, CDG = carbamoyl diphenylglycine, DPG = diphenylglycine, PYT = phenytoin. Impurity levels are given as weight %.

**Table 2: Stability Analysis at time = 0**

| **Example** | **pH** | **Recovery** | **RRT 0.33** | **CDG** | **DPG** | **RRT 0.47** | **PYT** | **Total Impurities** |
|---|---|---|---|---|---|---|---|---|
| Cerebyx | N/A | 98.1% | ND | 0.262% | ND | ND | 0.004% | 0.266% |
| C6 | 8.91 | 100.3% | ND | 0.004% | ND | ND | ND | 0.004% |
| C1 | 9.92 | 100.2% | ND | 0.003% | ND | ND | ND | 0.003% |
| C2 | 9.92 | 100.0% | ND | 0.004% | ND | ND | ND | 0.004% |
| C3 | 9.92 | 1002% | ND | 0.004% | ND | ND | ND | 0.004% |
| C4 | 9.94 | 100.4% | ND | 0.004% | ND | ND | ND | 0.004% |
| C5 | 9.91 | 100.5% | ND | 0.005% | ND | ND | ND | 0.005% |
| 1 | 7.97 | 99.6% | ND | 0.004% | ND | ND | ND | 0.004% |
| 2 | 7.98 | 99.5% | ND | 0.006% | ND | ND | ND | 0.006% |
| 3 | 7.94 | 98.6% | ND | 0.006% | ND | ND | ND | 0.006% |
| 4 | 8.00 | 97.8% | ND | 0.006% | ND | ND | ND | 0.006% |
| 5 | 8.03 | 96.5% | ND | 0.023% | ND | ND | ND | 0.023% |

**Table 3: Stability Analysis at 40°C/time = 2 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| C6 | 8.84 | 98.2% | ND | 0.338% | ND | ND | ND | 0.338% |
| C1 | 8.57 | 99.3% | 0.009% | 1.044% | 0.007% | ND | ND | 1.060% |
| C2 | 8.70 | 98.9% | 0.012% | 1.393% | 0.011% | ND | ND | 1.416% |
| C3 | 8.59 | 100.4% | ND | 0.930% | ND | ND | ND | 0.930% |
| C4 | 9.51 | 94.2% | 0.039% | 3.386% | 0.025% | ND | ND | 3.512% |
| C5 | 9.60 | 97.8% | ND | 2.743% | 0.023% | ND | ND | 2.827% |
| 1 | 7.77 | 99.1% | ND | 0.109% | ND | ND | ND | 0.109% |
| 2 | 7.81 | 98.8% | ND | 0.108% | ND | ND | ND | 0.108% |
| 3 | 7.79 | 98.8% | ND | 0.095% | ND | ND | ND | 0.095% |
| 4 | 7.93 | 99.1% | ND | 0.118% | ND | ND | ND | 0.118% |
| 5 | 7.98 | 98.5% | ND | 0.126% | ND | ND | ND | 0.126% |

**Table 4: Stability Analysis at 30°C/time = 2 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| C6 | 8.88 | 98.8% | ND | 0.147% | ND | ND | ND | 0.147% |
| C1 | 9.00 | 99.7% | 0.007% | 0.722% | 0.007% | ND | ND | 0.736% |
| C2 | 9.19 | 100.8% | ND | 0.902% | ND | ND | ND | 0.902% |
| C3 | 9.01 | 100.6% | ND | 0.606% | ND | ND | ND | 0.606% |
| C4 | 9.77 | 98.3% | 0.012% | 1.514% | ND | ND | ND | 1.526% |
| C5 | 9.77 | 99.9% | ND | 1.526% | ND | ND | ND | 1.526% |
| 1 | 7.82 | 99.3% | ND | 0.026% | ND | ND | ND | 0.026% |
| 2 | 7.83 | 98.9% | ND | 0.028% | ND | ND | ND | 0.028% |
| 3 | 7.80 | 98.3% | ND | 0.024% | ND | ND | ND | 0.024% |
| 4 | 7.93 | 99.2% | ND | 0.031% | ND | ND | ND | 0.031% |
| 5 | 8.01 | 98.8% | ND | 0.037% | ND | ND | ND | 0.037% |

**Table 5: Stability Analysis at 25 °C/time = 2 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| C6 | 8.90 | 99.3% | ND | 0.096% | ND | ND | ND | 0.096% |
| C1 | 9.33 | 99.2% | 0.007% | 0.548% | 0.008% | ND | ND | 0.563% |
| C2 | 9.49 | 101.1% | 0.005% | 0.650% | ND | ND | ND | 0.655% |
| C3 | 928 | 99.7% | 0.002% | 0.459% | ND | ND | ND | 0.461% |
| C4 | 9.87 | 100.4% | 0.007% | 0.965% | ND | ND | ND | 0.972% |
| C5 | 9.84 | 99.4% | ND | 1.072% | ND | ND | ND | 1.072% |
| 1 | 7.84 | 98.5% | ND | 0.015% | ND | ND | ND | 0.015% |
| 2 | 7.83 | 98.9% | ND | 0.014% | ND | ND | ND | 0.014% |
| 3 | 7.80 | 99.3% | ND | 0.012% | ND | ND | ND | 0.012% |
| 4 | 7.97 | 98.8% | ND | 0.016% | ND | ND | ND | 0.016% |
| 5 | 8.02 | 98.1% | ND | 0.020% | ND | ND | ND | 0.020% |

**Table 6: Stability Analysis at 40 °C/time = 4 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| Cerebyx | N/A | 97.9% | ND | 0.618% | 0.011% | ND | ND | 0.629% |
| C6 | 8.86 | 97.8% | ND | 0.636% | 0.007% | ND | ND | 0.643% |
| C1 | 8.39 | 97.4% | 0.021% | 1.262% | 0.023% | ND | ND | 1.306% |
| C2 | 8.45 | 96.5% | 0.052% | 1.679% | 0.037% | ND | ND | 1.783% |
| C3 | 8.42 | 98.0% | 0.018% | 1.153% | 0.020% | ND | ND | 1.191% |
| C4 | 9.31 | 89.5% | 0.376% | 4.810% | 0.077% | ND | 0.058% | 5.392% |
| C5 | 9.23 | 91.7% | 0.121% | 3.677% | 0.062% | ND | 0.175% | 4.189% |
| 1 | 7.87 | 100.5%% | ND | 0.202% | ND | ND | ND | 0.202% |
| 2 | 7.81 | 100.1% | ND | 0.201% | ND | ND | 0.101% | 0.302% |
| 3 | 7.79 | 100.7% | ND | 0.180% | ND | ND | 0.096% | 0.276% |
| 4 | 7.94 | 101.1% | ND | 0.219% | ND | ND | 0.065% | 0.284% |
| 5 | 7.91 | 100.5% | ND | 0.230% | ND | ND | 0.026% | 0.256% |

**Table 7: Stability Analysis at 30 °C/time = 4 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| Cerebyx | N/A | 97.3% | ND | 0.405% | ND | ND | ND | 0.405% |
| C6 | 8.86 | 98.6% | ND | 0.273% | 0.007% | ND | ND | 0.280% |
| C1 | 8.62 | 97.5% | 0.014% | 0.886% | 0.007% | ND | ND | 0.900% |
| C2 | 8.83 | 96.9% | 0.027% | 1.152% | 0.004% | ND | ND | 1.183% |
| C3 | 8.83 | 96.3% | 0.008% | 0.752% | ND | ND | ND | 0.760% |
| C4 | 9.53 | 95.2% | 0.118% | 2.457% | 0.005% | ND | ND | 2.580% |
| C5 | 9.55 | 96.4% | 0.036% | 2.405% | 0.009% | ND | ND | 2.450% |
| 1 | 7.89 | 101.1% | ND | 0.054% | ND | ND | ND | 0.054% |
| 2 | 7.86 | 99.4% | ND | 0.053% | ND | ND | ND | 0.053% |
| 3 | 7.79 | 100.1% | ND | 0.046% | ND | ND | 0.364%¹ | 0.410% |
| 4 | 7.96 | 101.2% | ND | 0.061% | ND | ND | ND | 0.061 % |
| 5 | 7.99 | 100.4% | ND | 0.072% | ND | ND | ND | 0.072% |

| | | | | | | | | |
|---|---|---|---|---|---|---|---|---|
| ¹Precipitation observed. | | | | | | | | |

**Table 8: Stability Analysis at 25 °C/time = 4 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| Cerebyx | N/A | 98.5% | ND | 0.356% | ND | ND | ND | 0.356% |
| C6 | 8.88 | 98.9% | ND | 0.181% | ND | ND | ND | 0.181% |
| C1 | 8.90 | 95.6% | 0.009% | 0.732% | ND | ND | ND | 0.741% |
| C2 | 9.12 | 94.6% | 0.018% | 0.889% | ND | ND | ND | 0.907% |
| C3 | 8.88 | 97.2% | 0.008% | 0.612% | ND | ND | ND | 0.620% |
| C4 | 9.67 | 87.6% | 0.053% | 1.412% | ND | ND | ND | 1.465% |
| C5 | 9.63 | 95.8% | 0.015% | 1.766% | ND | ND | ND | 1.782% |
| 1 | 7.91 | 102.0% | ND | 0.026% | ND | ND | ND | 0.026% |
| 2 | 7.89 | 100.8% | ND | 0.027% | ND | ND | ND. | 0.027% |
| 3 | 7.79 | 100.2% | ND | 0.023% | ND | ND | 0.271%¹ | 0.294% |
| 4 | 7.98 | 102.1 % | ND | 0.032% | ND | ND | ND | 0.032% |
| 5 | 8.01 | 101.6% | ND | 0.040% | ND | ND | ND | 0.040% |

| | | | | | | | | |
|---|---|---|---|---|---|---|---|---|
| ¹Precipitation observed. | | | | | | | | |

**Table 9: Stability Analysis at 40 °C/time = 6 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| C6 | 8.70 | 97.9% | ND | 0.951% | 0.021% | ND | ND | 0.972% |
| C1 | 8.22 | 95.3% | 0.023% | 1.428% | 0.038% | 0.015% | 0.051% | 1.555% |
| C3 | 8.25 | 96.4% | 0.028% | 1.324% | 0.039% | 0.013% | 0.047% | 1.451% |
| 1 | 7.70 | 100.2% | ND | 0.292% | 0.004% | ND | 0.177%¹ | 0.473°/a |
| 2 | 7.81 | 99.4% | ND | 0.296% | 0.004% | ND | 0.117%¹ | 0.417% |
| 3 | 7.79 | 100.0% | ND | 0.259% | ND | ND | 0.154%¹ | 0.413% |
| 4 | 7.91 | 99.7% | ND | 0.317% | 0.005% | ND | 0.122% | 0.444% |
| 5 | 7.94 | 99.3% | ND | 0.320% | 0.004% | ND | 0.120% | 0.444% |

| | | | | | | | | |
|---|---|---|---|---|---|---|---|---|
| ¹Precipitation observed. | | | | | | | | |

**Table 10: Stability Analysis at 30 °C/time = 6 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| C6 | 8.81 | 98.2% | ND | 0.423% | ND | ND | ND | 0.423% |
| C1 | 8.49 | 96.5% | 0.012% | 1.015% | 0.004% | 0.008% | ND | 1.039% |
| C3 | 8.39 | 96.9% | 0,012% | 0.891% | ND | 0.004% | ND | 0.907% |
| 1 | 7.77 | 98.2% | ND | 0.071% | ND | ND | 0.372%¹ | 0.443% |
| 2 | 7.84 | 100.7% | ND | 0.078% | ND | ND | ND | 0.078% |
| 3 | 7.80 | 99.6% | ND | 0.064% | ND | ND | 0.220%¹ | 0.284% |
| 4 | 7.96 | 100.1% | ND | 0.087% | ND | ND | ND | 0.087% |
| 5 | 8.00 | 99.4% | ND | 0.108% | ND | ND | ND | 0.108% |

| | | | | | | | | |
|---|---|---|---|---|---|---|---|---|
| ¹Precipitation observed. | | | | | | | | |

**Table 11: Stability Analysis at 25 °C/time = 6 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| C6 | 8.83 | 98.9% | ND | 0.273% | ND | ND | ND | 0.273% |
| C1 | 8.70 | 96.3% | 0.008% | 0.860% | ND | 0.007% | ND | 0.875% |
| C3 | 8.68 | 96.9% | 0.008% | 0.730% | ND | ND | ND | 0.739% |
| 1 | 7.82 | 99.4% | ND | 0.035% | ND | ND | 0.235%¹ | 0.270% |
| 2 | 7.85 | 99.3% | ND | 0.037% | ND | ND | 0.370%¹ | 0.407% |
| 3 | 7.81 | 98.9% | ND | 0.033% | ND | ND | 0.447%¹ | 0.480% |
| 4 | 7.99 | 99.6% | ND | 0.044% | ND | ND | ND | 0.044% |
| 5 | 8.04 | 100.2% | ND | 0.052% | ND | ND | ND | 0.052% |

| | | | | | | | | |
|---|---|---|---|---|---|---|---|---|
| ¹Precipitation observed. | | | | | | | | |

**Table 12: Stability Analysis at 40 °C/time = 8 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| Cerebyx | N/A | 98.4% | ND | 0.953% | 0.031% | ND | 0.110% | 1.094% |
| C6 | 8.74 | 100.3% | ND | 1.212% | 0.032% | ND | ND | 1.244% |
| C1 | 7.95 | 98.1% | 0.015% | 1.545% | 0.056% | 0.024% | 0.117% | 1.757% |
| C3 | 8.16 | 99.4% | 0.008% | 1.449% | 0.051% | 0.029% | 0.085% | 1.622% |
| 1 | 7.72 | 99.5% | 0.003% | 0.369% | 0.008% | ND | 0.176%¹ | 0.556% |
| 2 | 7.75 | 97.9% | 0.003% | 0.377% | 0.009% | ND | 0.153%¹ | 0.542% |
| 3 | 7.70 | 98.2% | ND | 0.329% | 0.006% | ND | 0.187%¹ | 0.522% |
| 4 | 7.83 | 98.8% | 0.003% | 0.404% | 0.010% | ND | 0.133% | 0.550% |
| 5 | 7.88 | 99.3% | 0.004% | 0.407% | 0.010% | ND | 0.141% | 0.562% |

| | | | | | | | | |
|---|---|---|---|---|---|---|---|---|
| ¹Precipitation observed. | | | | | | | | |

**Table 13: Stability Analysis at 30 °C/time = 8 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| Cerebyx | N/A | 98.1% | ND | 0.555% | 0.004% | ND | ND | 0.559% |
| C6 | 8.81 | 101.6% | ND | 0.554% | ND | ND | ND | 0.554% |
| C1 | 8.26 | 99.5% | 0.008% | 1.097% | 0.009% | 0.013% | ND | 1.127% |
| C3 | 8.36 | 100.5% | 0.004% | 0.982% | 0.008% | 0.014% | ND | 1.008% |
| 1 | 7.72 | 98.3% | ND | 0.092% | ND | ND | 0.155%¹ | 0.247% |
| 2 | 7.80 | 99.7% | ND | 0.101% | ND | ND | ND | 0.101% |
| 3 | 7.69 | 98.6% | ND | 0.081 % | ND | ND | 0.138%¹ | 0.219% |
| 4 | 7.89 | 100.4% | ND | 0.116% | ND | ND | ND | 0.116% |
| 5 | 7.98 | 99.2% | ND | 0.131% | ND | ND | ND | 0.131% |

| | | | | | | | | |
|---|---|---|---|---|---|---|---|---|
| ¹Precipitation observed. | | | | | | | | |

**Table 14: Stability Analysis at 25 °C/time = 8 weeks**

| **Example** | **pH** | **Recovery** | **RRT 0.33** | **CDG** | **DPG** | **RRT 0.47** | **PYT** | **Total Impurities** |
|---|---|---|---|---|---|---|---|---|
| Cerebyx | N/A | 100.1% | ND | 0.460% | ND | ND | ND | 0.460% |
| C6 | 8.86 | 100.8% | ND | 0.355% | ND | ND | ND | 0.355% |
| C1 | 8.50 | 100.0% | 0.005% | 0.928% | ND | 0.008% | ND | 0.936% |
| C3 | 8.57 | 101.2% | 0.002% | 0.807% | ND | 0.010% | ND | 0.819% |
| 1 | 7.70 | 98.2% | ND | 0.044% | ND | ND | 0.164%¹ | 0.208% |
| 2 | 7.80 | 98.0% | ND | 0.047% | ND | ND | 0.142%¹ | 0.189% |
| 3 | 7.72 | 98.4% | ND | 0.040% | ND | ND | 0.146%¹ | 0.186% |
| 4 | 7.88 | 99.4% | ND | 0.056% | ND | ND | ND | 0.056% |
| 5 | 8.00 | 99.6% | ND | 0.069% | ND | ND | ND | 0.069% |

| | | | | | | | | |
|---|---|---|---|---|---|---|---|---|
| ¹Precipitation observed. | | | | | | | | |

## Claims

1. An aqueous pharmaceutical composition comprising 5,5-diphenyl-3-[(phosphonooxy)methyl]-2,4-imidazolidinedione or a salt thereof, and a buffer selected from sodium bicarbonate, sodium phosphate, boric acid and glycine wherein said composition has a pH of less than 8.3.

2. A composition as claimed in claim 1 wherein said pH is greater than 7.5.

3. A composition as claimed in any one of claims 1 to 3 wherein said 5,5-diphenyl-3-[(phosphonooxy)methyl]-2,4-imidazolidinedione is present in the form of its disodium salt.

4. An aqueous pharmaceutical composition as defined in claim 1 for use in anti-convulsant treatment.

5. A syringe containing an aqueous pharmaceutical composition as defined in claim 1.

## Patentansprüche

1. Wässrige pharmazeutische Zusammensetzung, die 5,5-Diphenyl-3-[(phosphonooxy)methyl]-2,4-imidazolidindion oder ein Salz davon sowie einen aus Natriumbicarbonat, Natriumphosphat, Borsäure und Glycin ausgewählten Puffer umfasst, wobei die Zusammensetzung einen pH-Wert kleiner als 8,3 hat.

2. Zusammensetzung nach Anspruch 1, wobei der pH-Wert größer ist als 7,5.

3. Zusammensetzung nach einem der Ansprüche 1 bis 3, wobei das 5,5-Diphenyl-3-[(phosphonooxy)methyl]-2,4-imidazolidindion in Form seines Dinatriumsalzes vorliegt.

4. Wässrige pharmazeutische Zusammensetzung nach Anspruch 1 zur Verwendung bei einer krampflösenden Behandlung.

5. Spritze, die eine wässrige pharmazeutische Zusammensetzung nach Anspruch 1 enthält.

## Revendications

1. Composition pharmaceutique aqueuse comprenant de la 5,5-diphényl-3-[(phosphonooxy)méthyl]-2,4-imidazolidinedione ou un sel de celle-ci, et un tampon choisi parmi le bicarbonate de sodium, le phosphate de sodium, l'acide borique et la glycine, ladite composition ayant un pH inférieur à 8,3.

2. Composition selon la revendication 1, dans laquelle ledit pH est supérieur à 7,5.

3. Composition selon l'une quelconque des revendications 1 à 3, dans laquelle ladite 5,5-diphényl-3-[(phosphonooxy)méthyl]-2,4-imidazolidinedione est présente sous la forme de son sel de disodium.

4. Composition pharmaceutique aqueuse selon la revendication 1 pour son utilisation dans un traitement anticonvulsivant.

5. Seringue contenant une composition pharmaceutique aqueuse selon la revendication 1.
